(19) Europäisches Patentamt
European Patent Office
Office européen des brevets

(11) Veröffentlichungsnummer: **0 316 277**
**A2**

# (12) EUROPÄISCHE PATENTANMELDUNG

(21) Anmeldenummer: **88810750.5**

(22) Anmeldetag: **03.11.88**

(51) Int. Cl.[4]: **C 07 D 413/04**
C 07 D 209/14, B 41 M 5/00
// C07D209/12

(30) Priorität: **12.11.87 CH 4418/87**

(43) Veröffentlichungstag der Anmeldung:
**17.05.89 Patentblatt 89/20**

(84) Benannte Vertragsstaaten: **CH DE FR GB LI**

(71) Anmelder: **CIBA-GEIGY AG**
**Klybeckstrasse 141**
**CH-4002 Basel (CH)**

(72) Erfinder: **Phaff, Rox, Dr.**
**Dianastrasse 1**
**CH-4310 Rheinfelden (CH)**

## (54) Chromogene 1-heterocyclisch substituierte 2,4-Benzoxazine.

(57) Chromogene 1-heterocyclisch substituierte 2,4-Benzoxazine der Formel

(1) 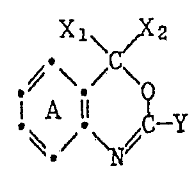

worin $X_1$ und $X_2$ gleiche oder voneinander verschiedene monocyclische oder polycyclische heteroaromatische Reste und $X_2$ auch einen Arylrest bedeuten,
Y für Wasserstoff, Alkyl, Cycloalkyl, Aryl, Aralkyl oder einen heterocyclischen Rest steht und der Benzolring A durch Halogen, Cyano, Nitro, Niederalkyl, Niederalkoxy, Niederalkylthio, Niederalkylcarbonyl, Niederalkoxycarbonyl oder $-NR_1R_2$ substituiert ist, wobei
$R_1$ und $R_2$, unabhängig voneinander, je Wasserstoff, unsubstituiertes oder durch Halogen, Hydroxy, Cyano oder Niederalkoxy substituiertes Alkyl mit höchstens 12 Kohlenstoffatomen, Cycloalkyl mit 5 bis 10 Kohlenstoffatomen oder unsubstituiertes oder durch Halogen, Cyano, Niederalkyl oder Niederalkoxy ringsubstituiertes Phenylalkyl oder Phenyl oder $R_1$ und $R_2$ zusammen mit dem sie verbindenden Stickstoffatom einen fünf- oder sechsgliedrigen, heterocyclischen Rest bedeuten.

Diese Benzoxazinverbindungen eignen sich insbesondere als Farbbildner in druck- oder wärmeempfindlichen Aufzeichnungsmaterialien und ergeben intensive und lichtechte orange, rosarote, rote, violette, grüne, grünblaue, violettblaue, graue oder schwarze Farbtöne.

EP 0 316 277 A2

**Beschreibung**

## Chromogene 1-heterocyclisch substituierte 2,4-Benzoxazine

Die vorliegende Erfindung betrifft chromogene 1-heterocyclisch substituierte 2,4-Benzoxazine, Verfahren zu ihrer Herstellung und ihre Verwendung als Farbbildner, in druckempfindlichen oder wärmeempfindlichen Aufzeichnungsmaterialien.

Die erfindungsgemässen chromogenen Verbindungen entsprechen der allgemeinen Formel

(1) 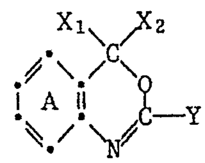

worin $X_1$ und $X_2$ gleiche oder voneinander verschiedene monocyclische oder polycyclische heteroaromatische Reste und $X_2$ auch einen Arylrest bedeuten,
Y für Wasserstoff, Alkyl, Cycloalkyl, Aryl, Aralkyl oder einen heterocyclischen Rest steht und der Benzolring A durch Halogen, Cyano, Nitro, Niederalkyl, Niederalkoxy, Niederalkylthio, Niederalkylcarbonyl, Niederalkoxycarbonyl oder $-NR_1R_2$ substituiert ist, wobei $R_1$ und $R_2$, unabhängig voneinander, je Wasserstoff, unsubstituiertes oder durch Halogen, Hydroxy, Cyano oder Niederalkoxy substituiertes Alkyl mit höchstens 12 Kohlenstoffatomen, Cycloalkyl mit 5 bis 10 Kohlenstoffatomen oder unsubstituiertes oder durch Halogen, Cyano, Niederalkyl oder Niederalkoxy ringsubstituiertes Phenalkyl oder Phenyl oder $R_1$ und $R_2$ zusammen mit dem sie verbindenden Stickstoffatom einen fünf- oder sechsgliedrigen, vorzugsweise gesättigten, heterocyclischen Rest bedeuten.

Niederalkyl, Niederalkoxy und Niederalkylthio stellen bei der Definition der Reste der Benzoxazine solche Gruppen oder Gruppenbestandteile dar, die 1 bis 5, insbesondere 1 bis 3 Kohlenstoffatome aufweisen. Beispiele für derartige Gruppen sind Methyl, Ethyl, n-Propyl, Isopropyl, n-Butyl, sek.- Butyl, Amyl oder Isoamyl bzw. Methoxy, Ethoxy, Isopropoxy, Isobutoxy oder tert.Butoxy bzw. Methylthio, Ethylthio, Propylthio oder Butylthio.

Halogen bedeutet beispielsweise Fluor, Brom oder vorzugsweise Chlor.

Die heteroaromatischen Reste $X_1$ und $X_2$ sind zweckmässigerweise über ein Kohlenstoffatom des Heteroringes an den Oxazinring gebunden.

Als derartige heteroaromatische Reste stellen $X_1$ und $X_2$ z.B. einen Pyrrolyl-, Thienyl-, Indolyl-, Benzofuranyl-, Benzothienyl- oder Naphthothienylrest dar.

Die ein- oder mehrkernigen heteroaromatischen Reste können einfach oder mehrfach ringsubstituiert sein. Als C-Substituenten kommen dabei z.B. Halogen, Hydroxyl, Cyano, Nitro, Niederalkyl, Niederalkoxy, Niederalkoxycarbonyl, Acyl mit 1 bis 8 Kohlenstoffatomen, vorzugsweise Niederalkylcarbonyl, Amino, Niederalkylamino oder Diniederalkylamino, $C_5$-$C_6$-Cycloalkyl, Benzyl oder Phenyl in Frage, während N-Substituenten beispielsweise $C_1$-$C_{12}$-Alkyl, $C_2$-$C_{12}$-Alkenyl, $C_1$-$C_8$-Acyl, Benzyl oder Phenethyl sind, die jeweils z.B. durch Cyano, Halogen, Nitro, Hydroxy, Niederalkyl, Niederalkoxy oder Niederalkoxycarbonyl substituiert sein können.

Die Alkyl- und Alkenylreste können geradkettig oder verzweigt sein. Beispiele hierfür sind Methyl, Ethyl, n-Propyl, Isopropyl, n-Butyl, t-Butyl, sek.Butyl, Amyl, n-Hexyl, 2-Ethyl-hexyl, Isooctyl, n-Octyl, Decyl oder n-Dodecyl bzw. Vinyl, Allyl, 2-Methylallyl, 2-Ethylallyl, 2-Butenyl oder Octenyl.

Acyl ist besonders Formyl, Niederalkylcarbonyl, wie z.B. Acetyl oder Propionyl, oder Benzoyl. Weitere Acylreste können Niederalkylsulfonyl, wie z.B. Methylsulfonyl oder Ethylsulfonyl sowie Phenylsulfonyl sein. Benzoyl und Phenylsulfonyl können durch Halogen, Methyl, Methoxy oder Ethoxy substituiert sein.

Bevorzugte heteroaromatische Reste sind substituierte 2-Pyrrolyl- oder vor allem 3-Indolylreste, wie z.B. N-$C_1$-$C_8$-Alkyl-pyrrol-2-yl- oder $C_1$-$C_8$-Alkyl-2-methylindol-3-ylreste. Diese Reste stehen besonders für $X_1$.

$X_2$ kann als Arylrest einen unsubstituierten oder durch Halogen, Cyano, Niederalkyl, Cycloalkyl, Acyl, Acylamino, $-NR_3R_4$, $-OR_3$ oder $-SR_3$ substituierten Phenyl- oder Naphthylrest bedeuten.

Vorzugsweise stellt $X_2$ einen substituierten Phenylrest der Formel

(1a) 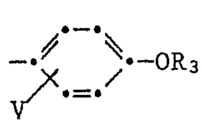 oder 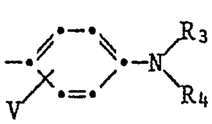 (1b)

dar.

$R_3$ und $R_4$ (unabhängig voneinander) haben je die für $R_1$ und $R_2$ angegebene Bedeutung.

In Formeln (1a) und (1b) bedeutet V Wasserstoff, Halogen, Niederalkyl, $C_1$-$C_{12}$-Alkoxy, $C_1$-$C_{12}$-Acyloxy, Benzyl, Phenyl, Benzyloxy, Phenyloxy, durch Halogen, Cyano, Niederalkyl oder Niederalkoxy substituiertes Benzyl oder Benzyloxy, oder die Gruppe -$NT_1T_2$. $T_1$ und $T_2$, unabhängig voneinander, bedeuten je Wasserstoff, Niederalkyl, Cycloalkyl, unsubstituiertes oder durch Halogen, Cyano, Niederalkyl oder Niederalkoxy substituiertes Benzyl, oder Acyl mit 1 bis 8 Kohlenstoffatomen und $T_1$ auch unsubstituiertes oder durch Halogen, Cyano, Niederalkyl oder Niederalkoxy substituiertes Phenyl. V befindet sich vorzugsweise in Ortho-Stellung zur Kohlenstoffbindung.

Stellen die Substituenten $R_1$, $R_2$, $R_3$, $R_4$ und Y Alkylgruppen dar, so können sie geradkettige oder verzweigte Alkylreste sein. Beispiele solcher Alkylreste sind Methyl, Ethyl, n-Propyl, Isopropyl, n-Butyl, sek.-Butyl, tert.-Butyl, Amyl, Isoamyl, n-Hexyl, 2-Ethyl-hexyl, n-Heptyl, n-Octyl, Isooctyl, n-Nonyl, Isononyl oder n-Dodecyl.

Sind die Alkylreste in $R_1$, $R_2$, $R_3$ und $R_4$ substituiert, so handelt es sich vor allem um Cyanoalkyl, Halogenalkyl, Hydroxyalkyl, Alkoxyalkyl jeweils vorzugsweise mit insgesamt 2 bis 6 Kohlenstoffatomen, wie z.B. β-Cyanoethyl, β-Chlorethyl, β-Hydroxyethyl, β-Methoxyethyl oder β-Ethoxyethyl.

Beispiele für Cycloalkyl in der Bedeutung der R-, Y- und T-Reste sind Cyclopentyl, Cycloheptyl oder vorzugsweise Cyclohexyl. Die Cycloalkylreste können einen oder mehrere $C_1$-$C_4$-Alkylreste, vorzugsweise Methylgruppen, enthalten und weisen insgesamt 5 bis 10 Kohlenstoffatome auf.

Der Acyloxyrest in V ist beispielsweise Formyloxy, Niederalkylcarbonyloxy oder Benzoyloxy. Als $C_1$-$C_{12}$-Alkoxyrest kann V eine geradkettige oder verzweigte Gruppe sein, wie z.B. Methoxy, Ethoxy, Isopropoxy, tert.Butoxy, n-Hexyloxy, Octyloxy oder Dodecyloxy.

Wenn die Substituentenpaare ($R_1$ und $R_2$) und ($R_3$ und $R_4$) zusammen mit dem gemeinsamen Stickstoffatom je einen heterocyclischen Rest darstellen, so ist dieser beispielsweise Pyrrolidino, Piperidino, Pipecolino, Morpholino, Thiomorpholino oder Piperazino, z.B. N-Methylpiperazino. Bevorzugte gesättigte heterocyclische Reste für -$NR_1R_2$ und -$NR_3R_4$ sind Pyrrolidino, Piperidino oder Morpholino.

Die Substituenten $R_1$, $R_2$, $R_3$ und $R_4$ sind vorzugsweise Cyclohexyl, Benzyl, Phenethyl, Cyano-Niederalkyl z.B. β-Cyanoethyl oder in erster Linie Niederalkyl, wie z.B. Methyl oder Ethyl. -$NR_1R_2$ und -$NR_3R_4$ sind bevorzugt auch Pyrrolidinyl.

V kann vorteilhafterweise Wasserstoff, Halogen, Niederalkyl, wie z.B. Methyl, Benzyloxy, $C_1$-$C_8$-Alkoxy, in erster Linie Niederalkoxy, wie z.B. Methoxy, Ethoxy, Isopropoxy oder tert.Butoxy, oder die Gruppe -$NT_1T_2$ sein, wobei von den Resten $T_1$ und $T_2$ eines vorzugsweise $C_1$-$C_8$-Acyl oder Niederalkyl und das andere Wasserstoff oder Niederalkyl ist. Der Acylrest ist in diesem Falle besonders Niederalkylcarbonyl, wie z.B. Acetyl oder Propionyl, Vorzugsweise ist V Acetylamino, Dimethylamino, Benzyloxy oder besonders Niederalkoxy und vor allem Ethoxy oder Wasserstoff.

Als heterocyclischer Rest stellt Y in erster Linie einen 5- oder 6-gliedrigen, vorzugsweise Sauerstoff, Schwefel oder Stickstoff aufweisenden Heterocyclus von aromatischem Charakter dar. Beispiele derartiger Heterocyclen sind Thienyl-, Furyl-, Pyrrolyl-, Pyrazolyl-, Imidazolyl-oder Pyridylreste.

Y ist vorzugsweise Phenyl oder durch Halogen, Trifluormethyl, Cyano, Niederalkyl oder Niederalkoxy substituiertes Phenyl.

Der Benzolring A kann 1 bis 3 Substituenten aufweisen. Zweckmässigerweise befinden sich die Substituenten in m-Stellung und/oder in p-Stellung zur Stickstoffbindung.

Der Ring A stellt vorzugsweise einen Benzolring dar, der in m-Stellung zur Stickstoffbindung einen Niederalkoxyrest oder den Rest -$NR_1R_2$ enthält. Dieser Benzolring A ist bevorzugt durch Halogen, Niederalkoxy oder Niederalkyl, vorzugsweise in p-Stellung zur Stickstoffbindung, weiter substituiert.

Praktisch wichtige heterocyclisch substituierte 2,4-Benzoxazine entsprechen der Formel

(2)

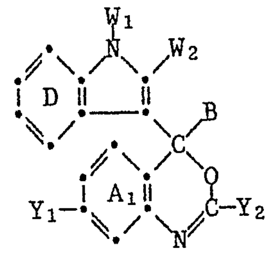

wobei B einen substituierten Phenylrest der Formel

(2a) 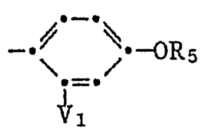 oder 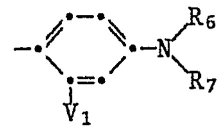 (2b)

$Y_1$ Niederalkoxy oder Diniederalkylamino,
$Y_2$ Phenyl oder durch Halogen, Trifluormethyl, Niederalkyl oder Niederalkoxy substituiertes Phenyl,
$W_1$ Wasserstoff, unsubstituiertes oder durch Cyano oder Niederalkoxy substituiertes $C_1$-$C_8$-Alkyl, Acetyl, Propionyl oder Benzyl,
$W_2$ Niederalkyl, vor allem Methyl oder Phenyl,
$R_5$, $R_6$ und $R_7$ unabhängig voneinander, je unsubstituiertes oder durch Hydroxy, Cyano oder Niederalkoxy substituiertes Alkyl mit höchstens 12 Kohlenstoffatomen, $C_5$-$C_6$-Cycloalkyl, Benzyl, Phenethyl oder Phenyl, oder ($R_6$ und $R_7$) zusammen mit dem sie verbindenden Stickstoffatom Pyrrolidino, Piperidino oder Morpholino,
$V_1$ Wasserstoff, Halogen, Niederalkyl, $C_1$-$C_8$-Alkoxy, Benzyloxy oder die Gruppe $-NT_3T_4$,
$T_3$ und $T_4$, unabhängig voneinander, je Wasserstoff, Niederalkyl, Niederalkylcarbonyl oder unsubstituiertes oder durch Halogen, Methyl oder Methoxy substituiertes Benzoyl bedeuten,
und die Ringe $A_1$ und D, unabhängig voneinander, unsubstituiert oder durch Halogen oder besonders Niederalkyl, wie Methyl substituiert sind.

Unter den Verbindungen der Formel (2) sind die Benzoxazine, in denen B einen substituierten Phenylrest der Formel (2a), $V_1$ Wasserstoff, $W_1$ $C_1$-$C_8$-Alkyl, $W_2$ Methyl und $Y_1$ Niederalkoxy bedeuten, bevorzugt.

Von besonderem Interesse sind Benzoxazine der Formel

(3) 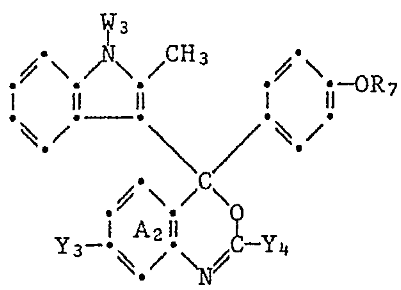

worin $A_2$ unsubstituiert oder vorzugsweise in p-Stellung zur Stickstoffbindung durch Halogen oder Methyl substituiert ist,
$Y_3$ Niederalkoxy, vor allem Methoxy,
$R_7$ und $W_3$, unabhängig voneinander, je Niederalkyl und
$Y_4$ Phenyl, Tolyl oder Chlorphenyl
bedeuten.

Die erfindungsgemässen Benzoxazine der Formeln (1) bis (3) werden dadurch hergestellt, dass man eine Methinverbindung der Formel

(4) 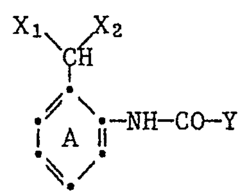

worin A, $X_1$, $X_2$ und Y die oben angegebene Bedeutung haben, oxidiert und cyclisiert.

Die Oxidation der Leukoverbindungen der Formel (4) zu den Benzoxazinverbindungen der Formel (1) erfolgt mit Oxidationsmitteln. Geeignete Oxidationsmittel sind z.B. Chromate, Bichromate, Chlorate, Chlorite, Nitrite, Perborate, Permanganate, Peroxide, z.B. Wasserstoffsuperoxid, Mangandioxid, Bleidioxid, molekularer Sauerstoff, Luft, Chloranil, Salze des Hexacyanoferrat-III wie Kalium- oder Natriumsalze oder Eisen-III-chloridhexahydrat.

Die Oxidation erfolgt bereits bei Raumtemperatur oder leicht erhöhter Temperatur (30 bis 50°C) vorzugsweise in organischen Lösungsmitteln, die gegebenenfalls mit Säuren verwendet werden können.

Geeignete Lösungsmittel, die das Reaktionsmedium bilden, sind Alkohole, wie z.B. Ethanol, Propanol, Ethylenglykolmonomethyl- oder -ethylether, Ketone, z.B. Aceton, Butanon oder Methylisopropylketon, Dimethylsulfoxid oder Nitrile aliphatischer Monocarbonsäuren, wie z.B. Acetonitril, Propionitril oder Butyronitril, cycloaliphatische oder aromatische Kohlenwasserstoffe, wie z.B. Cyclohexan, Benzol, Toluol oder Xylol; Chlorkohlenwasserstoffe, wie z.B. Ethylenchlorid, Tetrachlorethylen oder Chlorbenzole, wie z.B. Chlorbenzol, Chlortoluol oder Dichlorbenzol, cyclische Ether, wie z.B. Dioxan oder Tetrahydrofuran.

Als Säuren können beispielsweise Salzsäure, Schwefelsäure, Phosphorsäure, Essigsäure oder Propionsäure mitverwendet werden. Auch Mischungen der genannten Lösungsmittel und Säuren können eingesetzt werden.

Die Cyclisierung und die Isolierung des Endproduktes der Formel (1) erfolgt in allgemein bekannter Weise durch Einstellen des Oxidationsgemisches auf einen pH-Wert von mindestens 6, vorzugsweise 7 bis 11, z.B. mit Alkalien, wie z.B. Alkalimetallhydroxiden, Ammoniak, Alkalimetallcarbonaten oder -bicarbonaten und Abtrennen des gebildeten Niederschlags, Waschen und Trocknen oder durch Behandeln mit geeigneten organischen Lösungsmitteln, wie z.B. Methanol, Isopropanol, Benzol, Chlorbenzol, Toluol oder vor allem Propanol.

Die als Ausgangstoffe der Formel (4) verwendeten Leukoverbindungen können dadurch hergestellt werden, dass man eine Carbinolverbindung der Formel

(5) 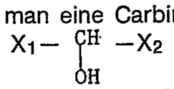

mit einer Aminoverbindung der Formel

(6) 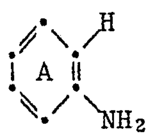

umsetzt und die erhaltene Methinverbindung der Formel

(7) 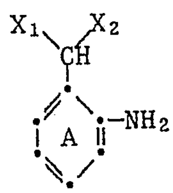

mit einer den Rest Y-CO- einführenden Verbindung auf übliche Weise acyliert, worin A, $X_1$, $X_2$ und Y die angegebenen Bedeutungen haben. Dabei stehen die Substituenten des Benzolringes A zweckmässigerweise in m-und/oder in p-Stellung zur Aminogruppe.

Die Umsetzung der Carbinolverbindung der Formel (5) mit der Aminoverbindung der Formel (6) erfolgt zweckmässig in einem polaren organischen Lösungsmittel, besonders in niederen aliphatischen Alkoholen, wie z.B. Methanol, Ethanol oder Isopropanol oder in Ethern, wie z.B. Tetrahydrofuran und vorzugsweise in Anwesenheit eines sauren Katalysators. Die Kondensation kann schon bei Raumtemperatur (20 bis 25°C) vorgenommen werden. Zweckmässig ist jedoch die Anwendung einer erhöhten Temperatur bis zur Rückflusstemperatur, vorzugsweise bei 40 bis 100°C. Als saure Katalysatoren eignen sich anorganische Säuren, wie z.B. Salzsäure, Schwefelsäure, Phosphorsäure oder Perchlorsäure, sowie auch niedere aliphatische Carbonsäuren, wie Ameisensäure oder Essigsäure.

Geeignete Acylierungsmittel zur Einführung des Acylrestes Y-CO- sind reaktionsfähige funktionelle Derivate von aliphatischen oder cycloaliphatischen Carbonsäuren, insbesondere Carbonsäurehalogenide oder -anhydride, wie z.B. Acetylbromid, Acetylchlorid oder Essigsäureanhydrid oder vor allem von aromatischen Carbonsäuren, wie z.B. gegebenenfalls ringsubstituierte Benzoesäurehalogenide.

Ein weiteres Verfahren zur Herstellung der Leukoverbindungen der Formel (4) besteht darin, dass man eine Amidverbindung der Formel

(8) 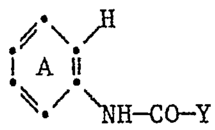

mit einer Ketonverbindung der Formel

(9) $X_1-CO-X_2$

umsetzt, worin A, $X_1$, $X_2$ und Y die oben angegebene Bedeutung haben.

Die Umsetzung erfolgt üblicherweise mit Wasser abspaltenden Reagenzien ohne oder auch in Anwesenheit von inerten organischen Lösungsmitteln bei einer Temperatur von der Raumtemperatur bis zum Siedepunkt des jeweiligen Mediums.

Die Benzoxazine der Formeln (1) bis (3) sind normalerweise farblos oder höchstens schwach gefärbt. Wenn diese Farbbildner mit einem vorzugsweise sauren Entwickler, d.h. einem Elektronenakzeptor, in Kontakt gebracht werden, so ergeben sie je nach Bedeutung von A, $X_1$ und $X_2$ und dem verwendeten Entwickler intensive orange, rosarote, rote, violette, grüne, grün-blaue, blaue, violett-blaue, graue oder schwarze Farbtöne, die sublimations- und besonders lichtecht sind. Die Benzoxazine der Formeln (1) bis (3) sind auch sehr wertvoll im Gemisch mit einem oder mehreren anderen bekannten Farbbildnern, z.B. 3,3-(Bis-aminophenyl-)-phthaliden, 3-Indolyl-3-aminophenylazaphthaliden, (3,3-Bis-indolyl-)-phthaliden, 3-Amino-fluoranen, 2,6-Diaminofluoranen, 2,6-Diamino-3-methyl-fluoranen, Leukoauraminen, Spiropyranen, Spirodipyranen, Chromenopyrazolen, Chromenoindolen, Phenoxazinen, Phenothiazinen, Chinazolinen, Rhodaminlaktamen, Carbazolylmethanen oder weiteren Triarylmethan-leukofarbstoffen, um blaue, marine-blaue, graue oder schwarze Färbungen zu ergeben.

Die Benzoxazine der Formeln (1) bis (3) zeigen sowohl auf aktivierten Tonen, wie auch auf phenolischen Unterlagen eine ausgezeichnete Farbintensität und vor allem Lichtechtheit. Sie eignen sich insbesondere als sich schnell entwickelnde Farbbildner für die Verwendung in einem wärmeempfindlichen oder insbesondere druckempfindlichen Aufzeichnungsmaterial, das sowohl Kopier- als auch Registriermaterial sein kann. Sie zeichnen sich dadurch aus, dass sie pH stabil und in den Kapselölen hervorragend löslich sind. Nach Belichtung in CB-Blatt weisen sie eine geringe Abnahme der Farbstärke (CB-Desaktivierung) auf.

Ein druckempfindliches Material besteht beispielsweise aus mindestens einem Paar von Blättern, die mindestens einen Farbbildner der Formeln (1) bis (3) gelöst in einem organischen Lösungsmittel und einen Elektronenakzeptor als Entwickler enthalten.

Typische Beispiele für solche Entwickler sind Aktivton-Substanzen, wie Attapulgus-Ton, Säureton, Bentonit, Montmorillonit, aktivierter Ton, wie z.B. säureaktiviertes Bentonit oder Montmorillonit, ferner Zeolith, Halloysit, Siliciumdioxyd, Aluminiumoxid, Aluminiumsulfat, Aluminiumphosphat, Zinkchlorid, Zinknitrat, aktiviertes Kaolin oder irgendein beliebiger Ton. Als Entwickler können auch sauer reagierende, organische Verbindungen, wie z.B. gegebenenfalls ringsubstituierte Phenole, Resorcine, Salicylsäuren, wie z.B. 3,5-Bis-($\alpha$,$\alpha$-dimethylbenzyl-)-salicylsäure oder 3,5-Bis-($\alpha$-methylbenzyl)-salicylsäure oder Salicylsäureester und deren Metallsalze, z.B. Zinksalze, sowie ein sauer reagierendes, polymeres Material, wie z.B. ein phenolisches Polymerisat, ein Alkyl phenolacetylenharz, ein Maleinsäure-Kolophonium-Harz oder ein teilweise oder vollständig hydrolysiertes Polymerisat von Maleinsäureanhydrid mit Styrol, Ethylen oder Vinylmethylether, oder Carboxymethylen verwendet werden. Es können auch Mischungen der genannten monomeren und polymeren Verbindungen eingesetzt werden. Besonders bevorzugte Entwickler sind säureaktiviertes Bentonit, Zinksalicylate oder die Kondensationsprodukte von p-substituierten Phenolen mit Formaldehyd. Die letzteren können auch mit Zink modifiziert sein.

Die Entwickler können zusätzlich auch im Gemisch mit an sich unreaktiven oder wenig reaktiven Pigmenten oder weiteren Hilfstoffen wie Kieselgel oder UV-Absorbern, wie z.B. 2-(2′-Hydroxyphenyl-)benztriazolen eingesetzt werden. Beispiele für solche Pigmente sind: Talk, Titandioxid, Aluminiumoxid, Aluminiumhydroxyd, Zinkoxid, Kreide, Tone wie Kaolin, sowie organische Pigmente, z.B. Harnstoff-Formaldehydkondensate (BET-Oberfläche 2-75 $m^2/g$) oder Melamin-Formaldehyd-Kondensationsprodukte.

Der Farbbildner liefert an den Punkten, an denen er mit dem Elektronenakzeptor in Kontakt kommt, eine gefärbte Markierung. Um eine frühzeitige Aktivierung der in dem druckempfindlichen Aufzeichnungsmaterial vorhandenen Farbbildner zu verhindern, werden diese in der Regel von dem Elektronenakzeptor getrennt. Dies kann zweckmässig erzielt werden, indem man die Farbbildner in schaum-, schwamm- oder bienenwabenartigen Strukturen einarbeitet. Vorzugsweise sind die Farbbildner in Mikrokapseln eingeschlossen, die sich in der Regel durch Druck zerbrechen lassen.

Beim Zerbrechen der Kapseln durch Druck, beispielsweise mittels eines Bleistiftes, wird die Farbbildnerlösung auf ein benachbartes mit einem Elektronenakzeptor beschichtetes Blatt übertragen, wodurch eine farbige Stelle erzeugt wird. Die Farbe resultiert aus dem dabei gebildeten Farbstoff, der im sichtbaren Bereich des elektromagnetischen Spektrums absorbiert.

Die Farbbildner werden vorzugsweise in Form von Lösungen in organischen Lösungsmitteln eingekapselt. Beispiele für geeignete Lösungsmittel sind vorzugsweise nichtflüchtige Lösungsmittel, z.B. polyhalogeniertes Paraffin oder Diphenyl, wie Chlorparaffin, Monochlordiphenyl oder Trichlordiphenyl, ferner Tricresylphosphat,

Di-n-butylphthalat, Dioctylphthalat, Trichlorbenzol, Trichlorethylphosphat, aromatische Ether, wie Benzylphenylether, Kohlenwasserstofföle, wie Paraffin oder Kerosin, z.B. mit Isopropyl, Isobutyl, sek-Butyl oder tert.-Butyl alkylierte Derivate von Diphenyl, Naphthalin oder Terphenyl, Dibenzyltoluol, partiell hydriertes Terphenyl, mono- bis tetra-$C_1$-$C_3$-alkylierte Diphenylalkane, Dodecylbenzol, benzylierte Xylole, oder weitere chlorierte oder hydrierte, kondensierte, aromatische Kohlenwasserstoffe. Oft werden Mischungen verschiedener Lösungsmittel, insbesondere Mischungen aus Paraffinölen oder Kerosin und Diisopropylnaphthalin oder partiell hydriertem Terphenyl, eingesetzt, um eine optimale Löslichkeit für die Farbbildung, eine rasche und intensive Färbung und eine für die Mikroverkapselung günstige Viskosität zu erreichen. Bei der Einkapselung zeichnen sich die erfindungsgemässen Benzoxazine dadurch aus, dass sie ausserordentlich hohe pH-Beständigkeit z.B. in einem pH-Bereich von 4 bis 10 zeigen.

Die Kapselwände können durch Koazervationskräfte gleichmässig um die Tröpfchen der Farbbildnerlösung herum gebildet werden, wobei das Einkapselungsmaterial z.B. in der US-Patentschrift 2,800,457 beschrieben ist. Die Kapseln können vorzugsweise auch aus einem Aminoplast oder modifizierten Aminoplasten durch Polykondensation gebildet werden, wie es in den britischen Patentschriften 989,264, 1,156,725, 1,301,052 und 1,355,124 beschrieben ist. Ebenfalls geeignet sind Mikrokapseln, welche durch Grenzflächenpolymerisation gebildet werden, wie z.B. Kapseln aus Polyester, Polycarbonat, Polysulfonamid, Polysulfonat, besonders aber aus Polyamid oder Polyurethan.

Die Farbbildner der Formeln (1) bis (3) enthaltenden Mikrokapseln können zur Herstellung von druckempfindlichen Kopiermaterialien der verschiedensten bekannten Arten verwendet werden. Die verschiedenen Systeme unterscheiden sich im wesentlichen voneinander durch die Anordnung der Kapseln, der Farbreaktanten und durch das Trägermaterial.

Bevorzugt wird eine Anordnung, bei der der eingekapselte Farbbildner in Form einer Schicht auf der Rückseite eines Uebertragungsblattes und der Elektronenakzeptor in Form einer Schicht auf der Vorderseite eines Empfangsblattes vorhanden sind.

Eine andere Anordnung der Bestandteile besteht darin, dass die den Farbbildner enthaltenden Mikrokapseln und der Entwickler in oder auf dem gleichen Blatt in Form einer oder mehrerer Einzelschichten oder in der Papierpulpe vorliegen.

Die Kapseln werden vorzugsweise mittels eines geeigneten Binders auf dem Träger befestigt. Da Papier das bevorzugte Trägermaterial ist, handelt es sich bei diesem Binder hauptsächlich um Papierbeschichtungsmittel, wie Gummi arabicum, Polyvinylalkohol, Hydroxymethylcellulose, Casein, Methylcellulose, Dextrin, Stärke, Stärkederivate oder Polymerlatices. Letztere sind beispielsweise Butadien-Styrolcopolymerisate oder Acrylhomo- oder -copolymere

Als Papier werden nicht nur normale Papiere aus Cellulosefasern, sondern auch Papiere, in denen die Cellulosefasern (teilweise oder vollständig) durch Fasern aus synthetischen Polymerisaten ersetzt sind, verwendet.

Die Verbindungen der Formeln (1) bis (3) können auch als Farbbildner in einem thermoreaktiven Aufzeichnungsmaterial verwendet werden. Dieses enthält in der Regel mindestens einen Schichtträger, einen Farbbildner, und einen Elektronenakzeptor und gegebenenfalls auch ein Bindemittel und/oder Wachs.

Thermoreaktive Aufzeichnungssysteme umfassen, z.B. wärmeempfindliche Aufzeichnungs- und Kopiermaterialien und -papiere. Diese Systeme werden beispielsweise zum Aufzeichnen von Informationen, z.B. in elektronischen Rechnern, Ferndruckern, Fernschreibern oder in Aufzeichnungsgeräten und Messinstrumenten, wie z.B. Elektrocardiographen, verwendet. Die Bilderzeugung (Markierung) kann auch manuell mit einer erhitzten Feder erfolgen. Eine weitere Einrichtung zur Erzeugung von Markierungen mittels Wärme sind Laserstrahlen.

Das thermoreaktive Aufzeichnungsmaterial kann so aufgebaut sein, dass der Farbbildner in einer Bindemittelschicht gelöst oder dispergiert ist und in einer zweiten Schicht der Entwickler in dem Bindemittel gelöst und dispergiert ist. Eine andere Möglichkeit besteht darin, dass sowohl der Farbbildner als auch der Entwickler in einer Schicht dispergiert sind. Das Bindemittel wird in spezifischen Bezirken mittels Wärme erweicht und an diesen Punkten, an denen Wärme angewendet wird, kommt der Farbbildner mit dem Elektronenakzeptor in Kontakt und es entwickelt sich sofort die erwünschte Farbe.

Als Entwickler eignen sich die gleichen Elektronenakzeptoren, wie sie in druckempfindlichen Papieren verwendet werden. Beispiele für Entwickler sind die bereits erwähnten Tonminerale und Phenolharze, oder auch phenolische Verbindungen, wie sie beispielsweise in der DE-PS 12,51,348 beschrieben sind, z.B. 4-tert.-Butylphenol, 4-Phenylphenol, Methylen-bis-(p-phenylphenol), 4-Hydroxydiphenylether, α-Naphthol, β-Naphthol, 4-Hydroxybenzoesäure-methylester oder -benzylester, 4-Hydroxydiphenylsulfon, 4′-Hydroxy-4-methyldiphenylsulfon, 4′-Hydroxy-4-isopropoxydiphenylsulfon, 4-Hydroxy-acetophenon, 2,2′-Dihydroxydiphenyl, 4,4′-Cyclohexylidendiphenol, 4,4′-Isopropylidendiphenol, 4,4′-Isopropyliden-bis-2-methylphenol), ein Antipyrinkomplex von Zinkthiocyanat, ein Pyridinkomplex von Zinkthiocyanat, 4,4-Bis-(4-hydroxyphenyl)valeriansäure, Hydrochinon, Pyrogallol, Phloroglucin, p-, m-, o-Hydroxybenzoesäure, Gallussäure, 1-Hydroxy-2-naphthoesäure sowie Borsäure oder organische, vorzugsweise aliphatische Dicarbonsäuren, wie z.B. Weinsäure, Oxalsäure, Maleinsäure, Zitronensäure, Citraconsäure oder Bernsteinsäure.

Vorzugsweise werden zur Herstellung des thermoreaktiven Aufzeichnungsmaterials schmelzbare, filmbildende Bindemittel verwendet. Diese Bindemittel sind normalerweise wasserlöslich, während die Benzoxazine und der Entwickler in Wasser schwer löslich oder unlöslich sind. Das Bindemittel sollte in der Lage sein, den Farbbildner und den Entwickler bei Raumtemperatur zu dispergieren und zu fixieren.

Bei Einwirkung von Wärme erweicht oder schmilzt das Bindemittel, so dass der Farbbildner mit dem Entwickler in Kontakt kommt und sich eine Farbe bilden kann. Wasserlösliche oder mindestens in Wasser quellbare Bindemittel sind z.B. hydrophile Polymerisate, wie Polyvinylalkohol, Polyacrylsäure, Hydroxyethylcellulose, Methylcellulose, Carboxymethylcellulose, Polyacrylamid, Polyvinylpyrrolidon, carboxylierte Butadien-Styrolcopolymerisate, Gelatine, Stärke oder veretherte Maisstärke.

Wenn der Farbbildner und der Entwickler in zwei getrennten Schichten vorliegen, können in Wasser unlösliche Bindemittel, d.h. in nichtpolaren oder nur schwach polaren Lösungsmitteln lösliche Bindemittel, wie z.B. Naturkautschuk, synthetischer Kautschuk, chlorierter Kautschuk, Alkydharze, Polystyrol, Styrol/Butadien-Mischpolymerisate, Polymethylacrylate, Ethylcellulose, Nitrocellulose und Polyvinylcarbazol, verwendet werden. Die bevorzugte Anordnung ist jedoch diejenige, bei der der Farbbildner und der Entwickler in einer Schicht in einem wasserlöslichen Bindemittel enthalten sind.

Die thermoreaktiven Schichten können weitere Zusätze enthalten. Zur Verbesserung des Weissgrades, zur Erleichterung des Bedruckens der Papiere und zur Verhinderung des Festklebens der erhitzten Feder können diese Schichten, z.B. Talk, Titandioxyd, Zinkoxyd, Aluminiumhydroxyd, Calciumcarbonat (z.B. Kreide), Tone oder auch organische Pigmente, wie z.B. Harnstoff-Formaldehydpolymerisate enthalten. Um zu bewirken, dass nur innnerhalb eines begrenzten Temperaturbereiches die Farbe gebildet wird, können Substanzen, wie Harnstoff, Thioharnstoff, Diphenylthioharnstoff, Acetamid, Acetanilid, Benzolsulfanilid, Stearinsäureamid, Phthalsäureanhydrid, Metallstearate, wie z.B. Zinkstearat, Phthalsäurenitril, Dimethylterephthalat oder andere entsprechende, schmelzbare Produkte, welche das gleichzeitige Schmelzen des Farbbildners und des Entwicklers induzieren, zugesetzt werden. Bevorzugt enthalten thermographische Aufzeichnungsmaterialien Wachse, z.B. Carnaubawachs, Montanawachs, Paraffinwachs, Polyethylenwachs, Kondensate höherer Fettsäureamide und Formaldehyde und Kondensate höherer Fettsäuren und Ethylendiamin.

Eine weitere Anwendung der Verbindungen der Formeln (1) bis (3) ist die Herstellung eines Farbbildes mittels photohärtbarer Mikrokapseln, wie sie z.B. in der DE-OS 3 247 488 beschrieben sind.

In den folgenden Beispielen beziehen sich die angegebenen Prozentsätze, wenn nichts anderes angegeben ist, auf das Gewicht.

Beispiel 1:

179,22 g Anissäuredimethylamid werden bei 20-25°C in 91,73 g Phosphoroxychlorid eingetragen. Dann werden 67,5 g 2-Methylindol zugegeben, worauf die Temperatur auf 45°C steigt. Sobald sie zu fallen beginnt, wird das Gemisch im Verlauf einer Stunde auf 100°C gebracht. Es wird auf 30°C abgekühlt und bei 30-60°C lässt man 178 g Natriumhydroxid in 500 ml Wasser zutropfen. Anschliessend werden weitere 500 ml Wasser zugegeben und das Gemisch 10 Stunden bei Raumtemperatur gerührt. Es wird abfiltriert, das erhaltene Granulat zerkleinert und 90 Minuten in 600 ml Ethanol aufgekocht. Hierauf wird auf 10°C gekühlt, filtriert und mit Ethanol gewaschen. Nach dem Trocknen erhält man 87,9 g einer rosafarbenen Ketoverbindung der Formel

(i) 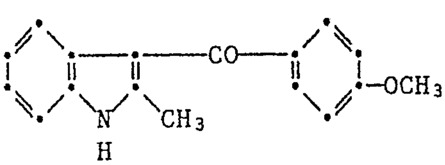

mit Smp. 212-213°C.

87,7 g obiger Verbindung werden bei 40-50°C in 500 ml Dimethylsulfoxid gelöst und auf Raumtemperatur gekühlt. Alsdann gibt man 124 ml einer 10 n wässrigen Kaliumhydroxidlösung zu und lässt während 30-45 Minuten 37 ml Ethylbromid zutropfen. Es wird 2 Stunden nachgerührt, auf 4 Liter Wasser ausgetragen und mit Methylenchlorid extrahiert. Die Behandlung mit Aktivkohle, Trocknung mit Natriumsulfat und das Einengen ergeben 92,6 g eines Oels, welches allmählich auskristallisiert. Umkristallisieren aus 2-Propanol ergibt 84,3 g einer Verbindung der Formel

(ii) 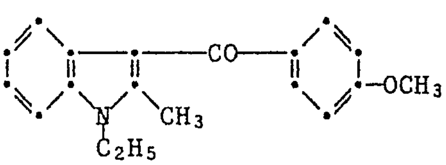

Smp. 104-105°C.

84,3 g der Verbindung der Formel (ii) werden in Tetrahydrofuran gelöst. Man lässt diese Lösung auf 5,44 g Lithiumaluminiumhydrid in 250 ml Tetrahydrofuran zutropfen. Nach 1 Stunde werden weitere 2,7 g

Lithiumaluminiumhydrid zugegeben. Nach 30 Minuten bei Raumtemperatur lässt man bei 5-10°C 15,3 ml Essigester und 8 ml Wasser zutropfen. Das Gemisch wird mit Natriumsulfat und Aktivkohle versetzt, dann filtriert und eingeengt. Man erhält 84,4 g einer Verbindung der Formel

(iii) 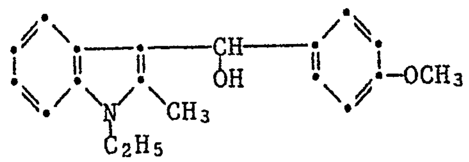

in Form eines Oeles.

84,4 g der Verbindung der Formel (iii) werden in 290 ml Methanol gelöst und mit 39,3 g 4-Amino-2-methoxytoluol versetzt. Alsdann werden 1,5 ml conc. Salzsäure zugegeben und 1 Stunde bei Rückfluss geheizt. Die entstandene Suspension wird auf 0°C gekühlt, filtriert und mit Methanol gewaschen. Man erhält 108,7 g einer Verbindung der Formel

(iv) 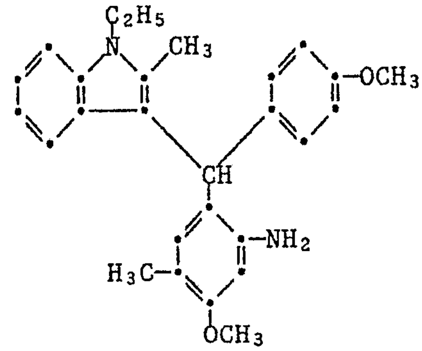

Smp. 171-172°C.

17,3 g der Verbindung der Formel (iv) werden in 42 ml Dichlorethan suspendiert und während 20-30 Minuten mit 5,9 g Benzoylchlorid in 18 ml Dichlorethan versetzt. Die resultierende Lösung wird 1 Stunde bei 30-40°C nachgerührt und anschliessend eingeengt. Der Rückstand wird in 63 ml Ethanol aufgenommen und mit 25 ml 10%iger Natriumhydroxidlösung versetzt. Die ausgefallene Substanz wird abfiltriert und getrocknet. Nach dem Umkristallisieren aus Toluol erhält man 14,3 g einer Verbindung der Formel

(v) 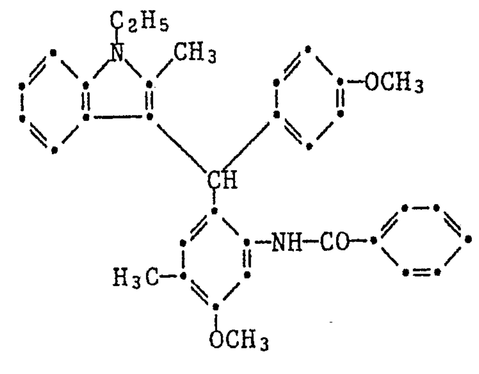

Smp. 207-208°C.

2,5 g der Verbindung der Formel (v) werden in 25 ml Aceton gelöst und mit 11,7 g Eisen-III-chlorid-hexahydrat versetzt. Nach 5 Stunden wird das Aceton abgezogen, der Rückstand in 50 ml Toluol aufgenommen und mit 80 ml 10%iger Natriumhydroxidlösung gewaschen. Die organische Phase wird abgetrennt, mit Aktivkohle behandelt, mit Natriumsulfat getrocknet und eingeengt. Der Rückstand wird aus 2-Propanol/Wasser umkristallisiert. Man erhält 1,57 g einer Verbindung der Formel

(11)

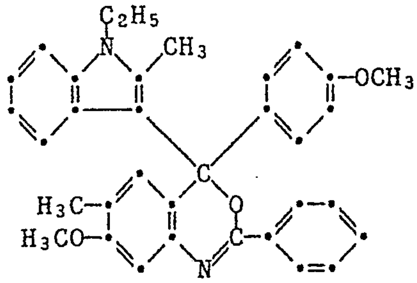

Smp. 137-147°C.

Diese Verbindung ergibt auf saurem Ton ein rosafarbenes Bild mit sehr guter Lichtechtheit.

Beispiel 2:

10,4 g der Verbindung der Formel (iv) gemäss Beispiel 1 werden in 25 ml Dichlorethan vorgelegt und während 45 Minuten bei 20-30°C mit einer Lösung von 4,4 g 4-Chlorbenzoylchlorid in 10 ml Dichlorethan versetzt. Es wird noch 1 Stunde bei 30-40°C nachgerührt, dann wird das Lösungsmittel abgezogen. Der Rückstand wird mit 37 ml Diethylether und 15 ml einer 10%igen Natriumhydroxidlösung verrührt, mit 400 ml Wasser verdünnt und mit Methylenchlorid extrahiert. Die organische Phase wird mit Wasser gewaschen, mit Aktivkohle behandelt, über Natriumsulfat getrocknet und eingeengt. Der Rückstand wird in Diethylether gelöst und mit Petroläther gefällt, worauf der Niederschlag abfiltriert und mit Petroläther gewaschen wird. Man erhält 9,0 g einer Leukoverbindung der Formel

(vi)

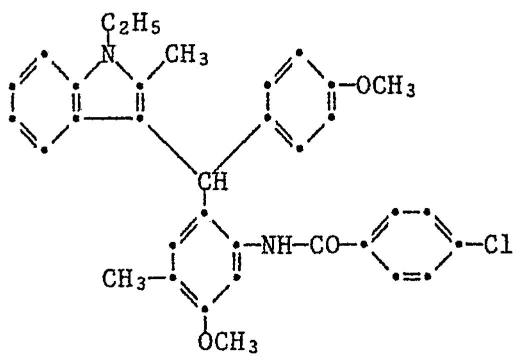

Smp. 164-165°C.

2,5 g der Verbindung der Formel (vi) werden in 25 ml Essigsäure gelöst und mit 6 g Eisen-III-chlorid-hexahydrat versetzt. Die Lösung wird 90 Minuten bei Raumtemperatur gerührt und dann auf 400 ml Wasser gegossen. Unter Eiskühlung wird mit 100 ml konz. Natriumhydroxidlösung basisch gestellt und mit Toluol extrahiert. Die organische Phase wird mit Aktivkohle behandelt, über Natriumsulfat getrocknet und eingeengt. Der Rückstand wird an Kieselgel chromatographiert (Hexan/Ether 2:1). Man erhält 0,6 g einer Benzoxazinverbindung der Formel

(12)

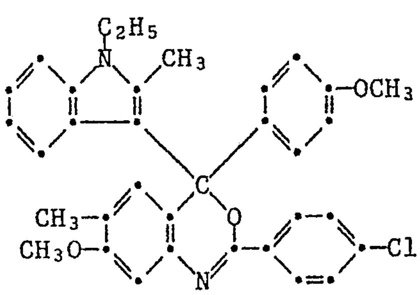

Smp. 120-122°C.

Auf saurem Ton ergibt diese Verbindung eine lichtechte rosa Färbung.

Beispiel 3:

6,5 g einer Verbindung der Formel

(vii) 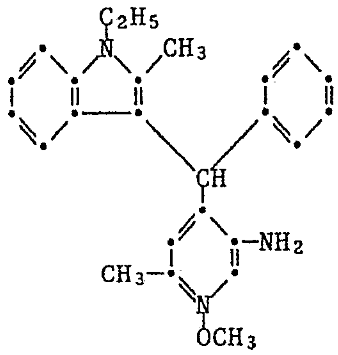

werden in 17 ml Dichlorethan suspendiert. Im Verlaufe von 45 Minuten lässt man bei 20-30°C 2,6 g Benzoylchlorid, gelöst in 7 ml Dichlorethan, zutropfen. Alsdann wird die Mischung 30 Minuten bei 30-35°C nachgerührt. Hierauf wird das Lösungsmittel im Vakuum abgezogen, der Rückstand in 25 ml Ethanol gelöst und die Lösung mit 10 ml 10 % Natriumhydroxidlösung versetzt. Das allmählich ausgefallene Produkt wird abfiltriert, mit Ethanol/Wasser (3:1) gewaschen und getrocknet. Man erhält 5,1 g einer Verbindung der Formel

(viii) 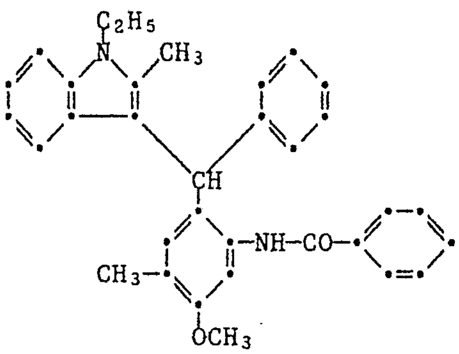

Smp. 170-171°C.

2,44 g der Verbindung der Formel (viii) werden in 25 ml Aceton gelöst und mit 10,8 g Eisen-III-chloridhexahydrat 14 Stunden bei Raumtemperatur verrührt. Das Lösungsmittel wird abgezogen, der Rückstand in Wasser aufgenommen und mit 20 ml konz. Natriumhydroxidlösung basisch gestellt. Das Gemisch wird mit Toluol extrahiert, mit Aktivkohle behandelt, filtriert und die organische Phase abgetrennt. Das Toluol wird abgezogen und der Rückstand aus 2-Propanol/Wasser umkristallisiert. Man erhält 1,7 g einer der Benzoxazinverbindung der Formel

(13) 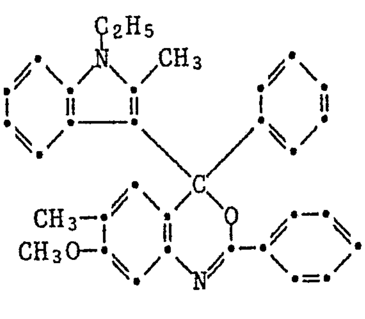

Smp. 168-169°C.

Diese Verbindung ergibt auf saurem Ton ein rosafarbenes Bild mit guter Lichtechtheit.

Beispiel 4

16,5 g (1-Ethyl-2-methylindol-3-yl)-(4-anisyl)-carbinol werden in 60 ml Methanol gelöst und mit 13,5 g 3-Dimethylaminobenzanilid versetzt. Das Gemisch wird auf 65°C gebracht, nach 15 Minuten mit 50 ml Methanol verdünnt und 12 Stunden bei 65°C gerührt. Dann wird abgekühlt, filtriert und mit Methanol gewaschen. Man erhält 43 g einer Verbindung der Formel

(ix) 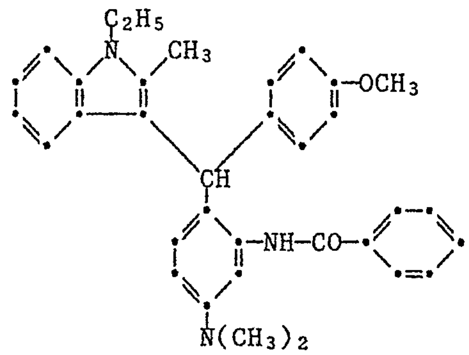

Smp. 178-178,5°C.

2,5 g der Verbindung der Formel (ix) werden in 25 ml Aceton gelöst und mit 10,4 g Eisen-III-chlorid-hexahydrat versetzt. Nach 20 Stunden bei Raumtemperatur wird das Reaktionsgemisch eingeengt, in Toluol gelöst und mit 80 ml 10 % Natriumhydroxidlösung behandelt. Die organische Phase wird abgetrennt, mit Aktivkohle behandelt, über Natriumsulfat getrocknet und eingeengt. Der Rückstand wird an Kieselgel chromatographiert. Man erhält 0,4 g einer Benzoxazinverbindung der Formel

(14) 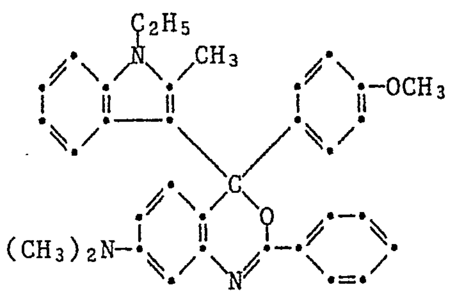

Smp. 119-124°C.

Auf saurem Ton ergibt die Benzoxazinverbindung ein blau-grünes Bild.

Auf gleiche Weise wie in den Beispielen 1 bis 4 beschrieben, erhält man unter Verwendung der entsprechenden Ausgangstoffe die in der folgenden Tabelle aufgeführten Benzoxazine der Formel

(15) 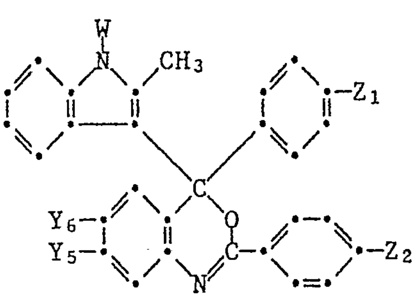

welche auf saurem Ton die angegebene Farbe ergeben.

Tabelle

| Bsp. | W | $Z_1$ | $Z_2$ | $Y_5$ | $Y_6$ | Farbe |
|---|---|---|---|---|---|---|
| 5 | $-CH_3$ | $-OC_2H_5$ | H | $-OCH_3$ | H | rosarot |
| 6 | $-C_2H_5$ | $-OCH_3$ | Cl | $-OCH_3$ | $-OCH_3$ | rot |
| 7 | $-C_8H_{17}$ | $-OCH_3$ | H | $-OCH_3$ | $CH_3$ | rosarot |
| 8 | $-C_2H_5$ | $-N(CH_3)_2$ | Cl | $-N(C_2H_5)_2$ | H | blauschwarz |
| 9 | $-C_8H_{17}$ | $-N(C_2H_5)_2$ | H | $-N(CH_3)_2$ | H | blauschwarz |
| 10 | $-C_2H_5$ | $-N(CH_3)_2$ | H | $-OCH_3$ | $CH_3$ | grünblau |
| 11 | $-C_2H_5$ | 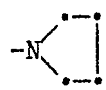 | H | $-N(CH_3)_2$ | H | schwarz |
| 12 | $-C_8H_{17}$ | 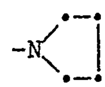 | H | $-OCH_3$ | $CH_3$ | grünblau |
| 13 | $-C_2H_5$ | 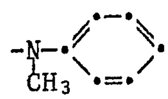 | Cl | $-OCH_3$ | $CH_3$ | grün |

Beispiel 14: Herstellung eines druckempfindlichen Kopierpapiers

Eine Lösung von 3 g des Benzoxazins der Formel (11) (Beispiel 1) in 80 g Diisopropylnaphthalin und 17 g Kerosin wird auf an sich bekannte Weise mit Gelatine und Gummi arabicum durch Koazervation mikroverkapselt, mit Stärkelösung vermischt und auf ein Blatt Papier gestrichen. Ein zweites Blatt Papier wird auf der Frontseite mit Aktivton als Farbentwickler beschichtet. Das erste den Farbbildner enthaltende Blatt und das mit Farbentwickler beschichtete Papier werden mit den Beschichtungen benachbart aufeinandergelegt. Durch Schreiben mit der Hand oder mit der Schreibmaschine auf dem ersten Blatt wird Druck ausgeübt, und es entwickelt sich sofort auf dem mit dem Entwickler beschichteten Blatt eine intensive rosarote Kopie, die ausgezeichnet lichtecht ist.

Beispiel 15:

1 g des Benzoxazins gemäss Beispiel 1 wird in 17 g Toluol gelöst. Zu dieser Lösung gibt man unter Rühren 12 g Polyvinylacetat, 8 g Calciumcarbonat und 2 g Titandioxid. Die erhaltene Suspension wird im Gewichtsverhältnis 1:1 mit Toluol verdünnt und mit einem 10 µm Rakel auf ein Blatt Papier gestrichen. Auf dieses Blatt Papier wird ein zweites Blatt Papier gelegt, dessen Unterseite bei einem Auftragsgewicht von 3 $g/m^2$ mit einer Mischung bestehend aus 1 Teil eines Amidwachses, 1 Teil eines Stearinwachses und 1 Teil Zinkchlorid beschichtet ist. Durch Schreiben mit der Hand oder mit der Schreibmaschine auf dem oberen Blatt wird Druck ausgeübt, und es entwickelt sich sofort auf dem mit dem Farbbildner beschichteten Blatt eine intensive und lichtechte rosarote Farbe.

Beispiel I6: Herstellung eines wärmeempfindlichen Aufzeichnungsmaterials

In einer Kugelmühle werden 32 g 4,4'Isopropylidendiphenol (Bisphenol A), 3,8 g Distearylamid des Ethylendiamins, 39 g Kaolin, 20 g eines zu 88 % hydrolysierten Polyvinylalkohols und 500 ml Wasser gemahlen bis die Teilchengrösse ca. 5 µm beträgt. In einer zweiten Kugelmühle werden 6 g des Benzoxazins gemäss Beispiel 1, 3 g eines zu 88 % hydrolysierten Polyvinylalkohols und 60 ml Wasser zu einer Teilchengrösse von ca. 3 µm gemahlen.

Die beiden Dispersionen werden zusammengegeben und mit einem Trockenauftragsgewicht von 5,5 $g/m^2$ auf ein Papier gestrichen. Durch Berührung des Papiers mit einem erhitzten Kugelschreiber wird eine intensive rosarote Farbe erhalten, die eine ausgezeichnete Licht-und Sublimierechtheit hat.

**Patentansprüche**

1. Chromogene 1-heterocyclisch substituierte 2,4-Benzoxazine der Formel

(1) 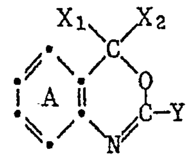

worin $X_1$ und $X_2$ gleiche oder voneinander verschiedene monocyclische oder polycyclische heteroaromatische Reste und $X_2$ auch einen Arylrest bedeuten,
Y für Wasserstoff, Alkyl, Cycloalkyl, Aryl, Aralkyl oder einen heterocyclischen Rest steht und der Benzolring A durch Halogen, Cyano, Nitro, Niederalkyl, Niederalkoxy, Niederalkylthio, Niederalkylcarbonyl, Niederalkoxycarbonyl oder $-NR_1R_2$ substituiert ist, wobei
$R_1$ und $R_2$, unabhängig voneinander, je Wasserstoff, unsubstituiertes oder durch Halogen, Hydroxy, Cyano oder Niederalkoxy substituiertes Alkyl mit höchstens 12 Kohlenstoffatomen, Cycloalkyl mit 5 bis 10 Kohlenstoffatomen oder unsubstituiertes oder durch Halogen, Cyano, Niederalkyl oder Niederalkoxy ringsubstituiertes Phenylalkyl oder Phenyl oder $R_1$ und $R_2$ zusammen mit dem sie verbindenden Stickstoffatom einen fünf- oder sechsgliedrigen, heterocyclischen Rest bedeuten.

2. Benzoxazine gemäss Anspruch 1, dadurch gekennzeichnet, dass $X_1$ und $X_2$, unabhängig voneinander, je einen Pyrrolyl-, Thienyl-, Indolyl-, Benzofuranyl-, Benzothienyl- oder Naphthothienylrest bedeuten.

3. Benzoxazine gemäss einem der Ansprüche 1 und 2, dadurch gekennzeichnet, dass in Formel (1) $X_1$ einen substituierten 2-Pyrrolyl- oder 3-Indolylrest darstellt.

4. Benzoxazine gemäss einem der Ansprüche 1 und 3, dadurch gekennzeichnet, dass in Formel (1) $X_2$ einen unsubstituierten oder durch Halogen, Cyano, Niederalkyl, Cycloalkyl, Acyl, Acylamino, $-NR_3R_4$, $-OR_3$ oder $-SR_3$ substituierten Phenyl- oder Naphthylrest bedeutet, worin $R_3$ und $R_4$, unabhängig voneinander, je Wasserstoff, unsubstituiertes oder durch Halogen, Hydroxy, Cyano oder Niederalkoxy substituiertes Alkyl mit höchstens 12 Kohlenstoffatomen, Cycloalkyl mit 5 bis 10 Kohlenstoffatomen oder unsubstituiertes oder durch Halogen, Cyano, Niederalkyl oder Niederalkoxy ringsubstituiertes Phenylalkyl oder Phenyl oder $R_3$ und $R_4$ zusammen mit dem sie verbindenden Stickstoffatom einen fünf- oder sechsgliedrigen, heterocyclischen Rest bedeuten.

5. Benzoxazine gemäss Anspruch 4, dadurch gekennzeichnet, dass in Formel (1) $X_2$ einen substituierten Phenylrest der Formel

(1a) 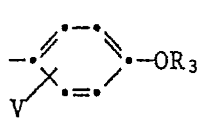 oder 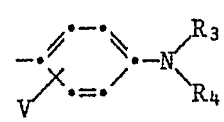 (1b)

worin $R_3$ und $R_4$, unabhängig voneinander, je Wasserstoff, unsubstituiertes oder durch Halogen, Hydroxy, Cyano oder Niederalkoxy substituiertes Alkyl mit höchstens 12 Kohlenstoffatomen, Cycloalkyl mit 5 bis 10 Kohlenstoffatomen oder unsubstituiertes oder durch Halogen, Cyano, Niederalkyl oder Niederalkoxy ringsubstituiertes Phenyalkyl oder Phenyl oder $R_3$ und $R_4$ zusammen mit dem sie verbindenden Stickstoffatom einen fünf- oder sechsgliedrigen, heterocyclischen Rest bedeuten und V Wasserstoff, Halogen, Niederalkyl, $C_1$-$C_{12}$-Alkoxy, $C_1$-$C_{12}$-Acyloxy, Benzyl, Phenyl, Benzyloxy, Phenyloxy, durch Halogen, Cyano, Niederalkyl oder Niederalkoxy substituiertes Benzyl oder Benzyloxy, oder die Gruppe $-NT_1T_2$, $T_1$ und $T_2$, unabhängig voneinander, je Wasserstoff, Niederalkyl, Cycloalkyl, unsubstituiertes oder durch Halogen, Cyano, Niederalkyl oder Niederalkoxy substituiertes Benzyl, oder Acyl mit 1 bis 8 Kohlenstoffatomen und $T_1$ auch unsubstituiertes oder durch Halogen, Cyano, Niederalkyl oder Niederalkoxy substituiertes Phenyl bedeuten.

6. Benzoxazine gemäss einem der Ansprüche 1 bis 5, dadurch gekennzeichnet, dass in Formel (1) Y Phenyl oder durch Halogen, Trifluormethyl, Cyano, Niederalkyl oder Niederalkoxy substituiertes Phenyl bedeutet.

7. Benzoxazine gemäss einem der Ansprüche 1 bis 6, dadurch gekennzeichnet, dass in Formel (1) der Ring A einen Benzolring darstellt, der in m-Stellung zur Stickstoffbindung durch Niederalkoxy oder durch $-NR_1R_2$ substituiert ist.

8. Benzoxazine gemäss Anspruch 7, dadurch gekennzeichnet, dass der Benzolring durch Halogen, Niederalkoxy oder Niederalkyl weiter substituiert ist.

9. Benzoxazine gemäss Anspruch 1, dadurch gekennzeichnet, dass sie der Formel

(2) 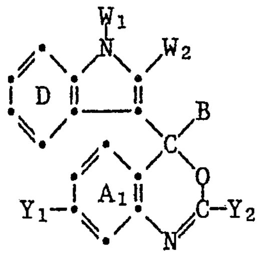

entsprechen, worin B einen substituierten Phenylrest der Formel

(2a) 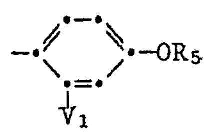 oder 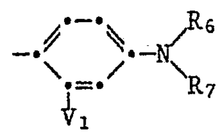 (2b)

$Y_1$ Niederalkoxy oder Diniederalkylamino,
$Y_2$ Phenyl oder durch Halogen, Trifluormethyl, Niederalkyl oder Niederalkoxy substituiertes Phenyl,
$W_1$ Wasserstoff, unsubstituiertes oder durch Cyano oder Niederalkoxy substituiertes $C_1$-$C_8$-Alkyl, Acetyl, Propionyl oder Benzyl,
$W_2$ Niederalkyl oder Phenyl,
$R_5$, $R_6$ und $R_7$, unabhängig voneinander, je unsubstituiertes oder durch Hydroxy, Cyano oder Niederalkoxy substituiertes Alkyl mit höchstens 12 Kohlenstoffatomen, $C_5$-$C_6$-Cycloalkyl, Benzyl, Phenethyl oder Phenyl, oder ($R_6$ und $R_7$) zusammen mit dem sie verbindenden Stickstoffatom Pyrrolidino, Piperidino oder Morpholino,
$V_1$ Wasserstoff, Halogen, Niederalkyl, $C_1$-$C_8$-Alkoxy, Benzyloxy oder die Gruppe -$NT_3T_4$,
$T_3$ und $T_4$, unabhängig voneinander, je Wasserstoff, Niederalkyl, Niederalkylcarbonyl oder unsubstituiertes oder durch Halogen, Methyl oder Methoxy substituiertes Benzoyl bedeuten,
und die Ringe $A_1$ und D, unabhängig voneinander, unsubstituiert oder durch Halogen oder Niederalkyl substituiert sind.

10. Benzoxazine gemäss Anspruch 9, dadurch gekennzeichnet, dass in Formel (2) B einen substituierten Phenylrest der Formel (2a), $V_1$ Wasserstoff, $W_1$ $C_1$-$C_8$-Alkyl, $W_2$ Methyl und $Y_1$ Niederalkoxy bedeuten.

11. Benzoxazine gemäss Anspruch 1, dadurch gekennzeichnet, dass sie der Formel

(3) 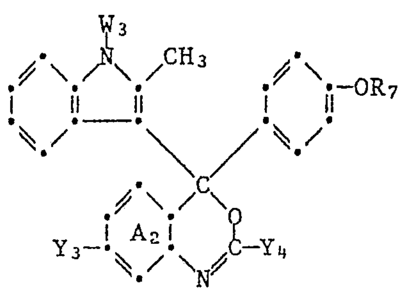

entsprechen, worin $A_2$ unsubstituiert oder durch Halogen oder Methyl substituiert ist,
$Y_3$ Niederalkoxy,
$R_7$ und $W_3$, unabhängig voneinander, je Niederalkyl und
$Y_4$ Phenyl, Tolyl oder Chlorphenyl bedeuten.

12. Verfahren zur Herstellung von 1-heterocyclisch substituierten 2,4-Benzoxazinen der Formel (1), dadurch gekennzeichnet, dass man eine Methinverbindung der Formel

(4) 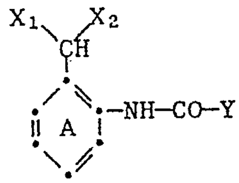

worin A, $X_1$, $X_2$ und Y die in Anspruch 1 angegebene Bedeutung haben, oxidiert und cyclisiert.

13. Verwendung einer 1-heterocyclisch substituierten 2,4-Benzoxazinverbindung der in einem der Ansprüche 1 bis 11 angegebenen Formel als Farbbildner zur Herstellung eines druckempfindlichen oder wärmeempfindlichen Aufzeichnungsmaterials.

14. Leukoverbindungen der Formel

(4) 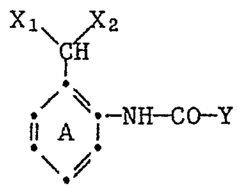

worin A, $X_1$, $X_2$ und Y die in Anspruch 1 angegebene Bedeutung haben.

15. Verfahren zur Herstellung von Leukoverbindungen der Formel (4), dadurch gekennzeichnet, dass man eine Carbinolverbindung der Formel

(5) $X_1$- CH -$X_2$
OH

mit einer Aminoverbindung der Formel

(6) 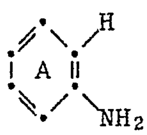

umsetzt und die erhaltene Methinverbindung der Formel

(7) 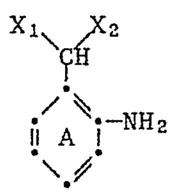

mit einer den Rest Y-CO- einführenden Verbindung auf übliche Weise acyliert, worin A, $X_1$, $X_2$ und Y die in Anspruch 1 angegebene Bedeutung haben.

16. Verfahren zur Herstellung von Leukoverbindungen der Formel (4) dadurch gekennzeichnet, dass man eine Amidverbindung der Formel

(8) 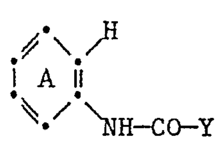

mit einer Ketoverbindung der Formel

(9) $X_1$-CO-$X_2$

umsetzt, worin A, $X_1$, $X_2$ und Y die in Anspruch 1 angegebene Bedeutung haben.

17. Druck- oder wärmeempfindliches Aufzeichnungsmaterial, dadurch gekennzeichnet, dass es in seinem Farbreaktantensystem als Farbbildner mindestens eine Benzoxazinverbindung der in einem der Ansprüche 1 bis 11 angegebenen Formel enthält.

18. Druckempfindliches Aufzeichnungsmaterial gemäss Anspruch 17, dadurch gekennzeichnet, dass es die Benzoxazinverbindung, gelöst in einem organischen Lösungsmittel, und mindestens einen festen Elektronenakzeptor enthält.

19. Druckempfindliches Aufzeichnungsmaterial gemäss einem der Ansprüche 17 und 18, dadurch gekennzeichnet, dass die Benzoxazinverbindung in Mikrokapseln eingekapselt ist.

20. Druckempfindliches Aufzeichnungsmaterial gemäss Anspruch 19, dadurch gekennzeichnet, dass die eingekapselte Benzoxazinverbindung in Form einer Schicht auf der Rückseite eines Uebertragungsblattes und der Elektronenakzeptor in Form einer Schicht auf der Vorderseite des Empfangsblattes vorhanden sind.

21. Druckempfindliches Aufzeichnungsmaterial gemäss einem der Ansprüche 17 bis 20, dadurch gekennzeichnet, dass die Benzoxazinverbindung gemeinsam mit einem oder mehreren anderen Farbbildnern enthalten ist.

22. Wärmeempfindliches Aufzeichnungsmaterial gemäss Anspruch 17, dadurch gekennzeichnet, dass es in mindestens einer Schicht mindestens eine Benzoxazinverbindung der in einem der Ansprüche 1 bis 11 angegebenen Formel, einen Elektronenakzeptor und gegebenenfalls ein Bindemittel und/oder Wachs enthält.